# FASCICULE DE BREVET EUROPEEN

(11) **EP 0 549 458 B1**
(45) Date de publication et mention de la délivrance du brevet: **02.06.1999**
(21) Numéro de dépôt: 92403515.7
(22) Date de dépôt: 22.12.1992
(51) Int. Cl.: A61B 17/22

(54) **Dispositif rotatif d'athérectomie ou de thrombectomie**
Vorrichtung zur Atherektomie oder Thrombektomie
Rotating instrument for atherectomy or thrombectomy

(30) Priorité: 23.12.1991 FR 9116422
(43) Date de publication de la demande: 30.06.1993
(73) Titulaire: LEFEBVRE, Jean-Marie, F-59800 Lille (FR)
(72) Inventeur: LEFEBVRE, Jean-Marie, F-59800 Lille (FR)
(74) Mandataire: Bruin, Cornelis Willem

(56) Documents cités:
- EP-A- 0 014 150
- EP-A- 0 308 957
- EP-A- 0 315 290
- EP-A- 0 379 784
- EP-A- 0 419 154
- EP-A- 0 426 322
- US-A- 5 030 201

## Description

La présente invention concerne un dispositif d'athérectomie ou de thrombectomie, comme caracterisé au préambule de la revendication 1.

Un dispositif de ce type est connu de US-A-5.030.201.

Ce dispositif comporte un système de rapprochement axial des extrémités proximales et distales, comprenant un élément de traction sur lequel peut agir l'opérateur et qui permet de rapprocher l'extrémité distale de l'outil rotatif vers l'extrémité proximale des éléments longiformes flexibles. Lors de ce rapprochement, chaque élément longiforme se recourbe, ce qui conduit à un développement transversal de l'outil rotatif. Les éléments longiformes flexibles sont réalisés en acier.

Le dispositif décrit dans le document US-A-5.030.201 est de réalisation relativement complexe, en particulier compte tenu de la présence des moyens de rapprochement axial des deux extrémités proximales et distales des éléments longiformes, constituant l'outil rotatif.

Le but visé par le demandeur est de proposer un dispositif rotatif d'athérectomie qui pallie l'inconvénient précité du dispositif décrit dans les documents précités.

Ce but est parfaitement atteint par le dispositif de l'invention comme caracterisé en revendication 1.

De manière caractéristique, selon l'invention, le dispositif est exempt de moyens mécaniques de rapprochement desdites extrémités; de plus la flexibilité des éléments longiformes est suffisante pour permettre leur développement transversal sous l'effet de la seule force centrifuge.

Ainsi c'est la commande de la vitesse de rotation de l'outil qui permet à l'opérateur d'agir sur le diamètre d'attaque de l'outil à l'intérieur du vaisseau.

On comprend que pour une vitesse de rotation donnée de l'organe rotatif, l'importance du développement transversal sera fonction de la longueur des éléments longiformes ainsi que de leur flexibilité.

Par ailleurs, selon les constatations du demandeur, le développement transversal de l'organe rotatif, sous le seul effet de la force centrifuge, procure au dispositif de l'invention une meilleure adaptabilité du diamètre d'attaque en fonction des variations de diamètre interne du vaisseau.

Les éléments longiformes flexibles sont constitués par les bandes formées dans la canalisation entre chaque entaille, et en consequence sont de materieau plastique de la canalisation. On comprend que lesdites entailles ne sont pas prolongées jusqu'à l'extrémité de la canalisation mais s'arrêtent avant celle-ci de telle sorte que les bandes restent solidaires par cette extrémité. Comme on peut le comprendre, cette version du dispositif selon l'invention est d'une réalisation tout à fait simple.

Avantageusement, le dispositif d'athérectomie de l'invention comporte un fil de guidage, rigide, destiné à être enfilé dans la canalisation et prolongeant l'extrémité avant de celle-ci d'une longueur prédéterminée.

Pour assurer le bon fonctionnement du dispositif de l'invention, la longueur du fil de guidage, au delà de l'extrémité avant de la canalisation, doit être suffisante pour assurer l'appui dudit fil sur la paroi interne du vaisseau, de telle manière que lors de la rotation de l'organe rotatif, il ne se produise pas un effet de battement du fil de guidage contre la paroi du vaisseau. On comprend que cette longueur predéterminée sera fonction du diamètre du vaisseau et également de la rectitude de celui-ci dans la zone où se trouve le dépôt à éliminer.

La canalisation est par exemple réalisée en polytétrafluoréthylène, qui est un matériau qui d'une part a une flexibilité suffisante pour réaliser le développement transversal des bandes flexibles prédécoupées sous le seul effet de la force centrifuge et d'autre part a un très faible coefficient de frottement en sorte que le glissement de l'extrémité avant de la canalisation le long du fil de guidage, lors de la mise en rotation de la canalisation entraîne très peu de perte d'énergie.

Selon une variante de réalisation, les éléments longiformes flexibles de l'organe rotatif ont une surface abrasive. Cette version est particulièrement utile lorsqu'il s'agit d'athérectomie artérielle, pour laquelle il est nécessaire de pulvériser les dépôts en très fines particules.

Pour obtenir ce caractère abrasif, il est possible soit d'inclure de la poudre abrasive dans le matériau plastique constitutif de la canalisation, au moins dans sa partie avant correspondant à l'outil rotatif, soit de coller cette poudre abrasive en surface des éléments longiformes flexibles.

De préférence selon le dispositif de l'invention, l'organe rotatif est composé de deux jeux successifs d'éléments longiformes flexibles. Ainsi, il est possible d'obtenir le développement transversal d'un organe rotatif composé de deux outils, placés à une distance déterminée l'un de l'autre dans le vaisseau. Cette possibilité était totalement exclue lorsque le développement transversal de l'outil rotatif était obtenu par des moyens mécaniques de rapprochement des extrémités proximales et distales des éléments longiformes souples constituant ledit outil.

Selon le demandeur, cette disposition particulière permet d'adapter au mieux la progressivité de l'attaque des dépôts notamment lorsque la longueur des éléments longiformes du jeu le plus distal est inférieure à celle des éléments longiformes du jeu le plus proximal. Dans ce cas, pour une même vitesse de rotation de l'organe rotatif, on a d'abord l'action du premier jeu d'éléments longiformes suivant un diamètre d'attaque plus réduit, puis l'action du deuxième jeu d'éléments longiformes selon un diamètre d'attaque plus important. Ceci permet de désobstruer et d'éliminer les dépôts dans des vaisseaux de gros diamètres, en particulier la veine cave.

L'invention sera mieux comprise à la lecture de la description qui va maintenant être faite de deux exemples de réalisation d'un dispositif rotatif d'athérectomie à développement transversal centrifuge, illustré par le dessin annexé dans lequel :
Les figures 1A et 1B sont une vue de côté du dispositif, de son cathéter d'introduction, et du fil de guidage, en position développée (Fig. 1A) et en position d'introduction (Fig. 1B)
La figure 2 est une vue en coupe d'un dispositif à deux outils rotatifs à l'intérieur d'un vaisseau.

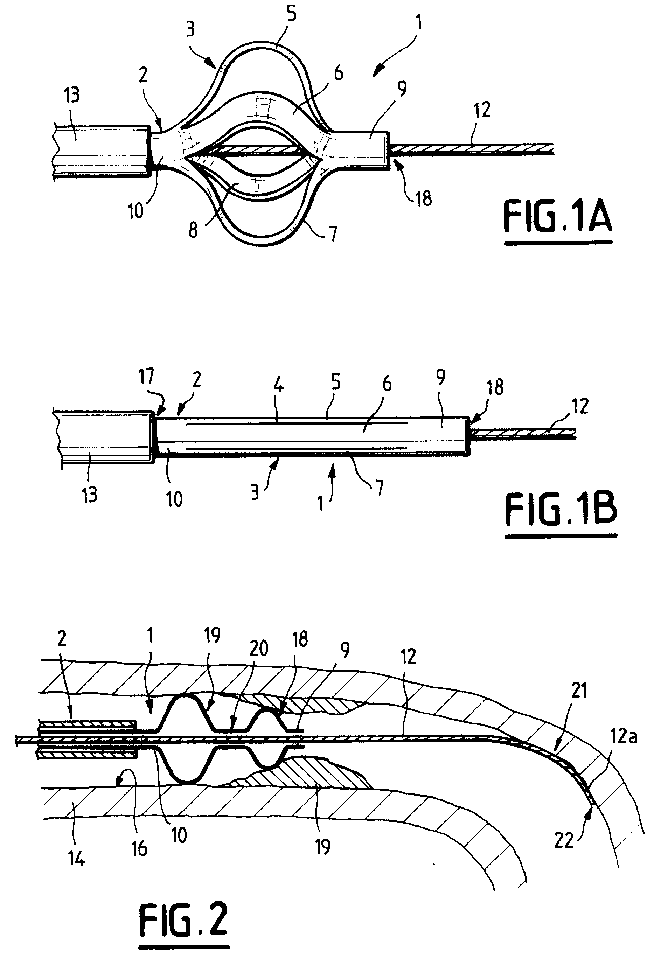

On n'a représenté à la figure 1 que la partie avant du dispositif d'athérectomie selon l'invention. Il s'agit en fait de la partie active du dispositif, c'est-à-dire celle qui lors de sa rotation permet de détruire les dépôts anormaux adhérant à la paroi interne d'un vaisseau sanguin. De tels dépôts sont, comme on le sait, une cause importante de maladies cardiovasculaires, lorsqu'il s'agit de dépôts intra-artériels d'athérome, ou plaques athérosclérotiques. Un tel dépôt peut aussi provoquer une altération de la fonction de certains organes.

Le dispositif 1 d'athérectomie selon l'invention comporte une canalisation 2 dont la partie avant 3 présente sur une partie de sa longueur et longitudinalement des entailles 4 régulièrement réparties sur sa périphérie. Dans l'exemple montré à la figure 1, il s'agit de quatre entailles 4 délimitant quatre bandes 5, 6, 7, 8 de même largeur et de même longueur. Les entailles 4 ne vont pas jusqu'à l'extrémité avant 8 de la canalisation 2, de telle sorte que les quatre bandes 5 - 8 restent solidaires l'une de l'autre grâce à l'extrémité distale 9 de la canalisation 2.

L'extrémité proximale de la canalisation 2, qui n'est pas visible sur les figures 1 et 2, est assortie de moyens d'entraînement en rotation. De tels moyens sont connus en eux-mêmes, il peut s'agir de moyens d'entraînement mécaniques tels que ceux décrits dans le document EP 0086048 ou encore et de préférence de moyens d'entraînement pneumatiques tels que ceux décrits dans le document EP 0268228.

Ces moyens pneumatiques comprennent une turbine munie de pales, dont la rotation est obtenue par l'alimentation tangentielle d'un fluide comprimé. Cette turbine est traversée par une tubulure axiale dans laquelle est montée à force l'extrémité proximale de la canalisation 2.

Une telle turbine permet d'obtenir des vitesses de rotation très importantes de l'ordre de 200 000 tours par minute.

Sur la figure 1B est représentée la partie avant 3 de la canalisation 2 en position normale, c'est-à-dire lorsque la canalisation n'est pas entraînée en rotation par la turbine ou par tout autre moyen d'entraînement. Les bandes 5 - 8 sont rectilignes longitudinalement dans le prolongement normal de la canalisation 2 et de l'extrémité distale 9.

Lors de la mise en rotation de la canalisation 2 et plus particulièrement lorsque celle-ci atteint une certaine vitesse, il se produit un développement transversal progressif des bandes 5 - 8, sous l'effet de la force centrifuge due à cette rotation. Ce développement transversal entraîne le rapprochement de l'extrémité distale 9 de la canalisation 2 vers l'autre extrémité proximale 10 de la partie avant 3 de la canalisation 2.

S'agissant d'une canalisation réalisée en polytétrafluoréthylène, ayant un diamètre compris entre 1,5 et 2 mm, chaque bande 5 - 8 ayant une longueur d comprise entre 1 et 2 cm, on a obtenu un développement transversal des bandes à partir d'une vitesse de rotation de l'ordre de 30 000 tours/mn avec un développement transversal maximum à 150 000 tours/mn.

La mise en oeuvre du dispositif 1 nécessite deux éléments complémentaires à savoir d'une part le fil de guidage 12 et d'autre part le cathéter d'introduction 13.

Le fil de guidage 12 est un fil qui doit être à la fois flexible pour suivre le trajet éventuellement sinueux du vaisseau, et rigide pour pouvoir assurer le maintien et le guidage du dispositif rotatif d'athérectomie de l'invention. Son extrémité distale est généralement dans un matériau radio-opaque permettant de localiser facilement par radioscopie l'emplacement du fil de guidage.

Le cathéter 13 est d'un type connu. Son diamètre intérieur est suffisant pour recevoir la canalisation 2.

La mise en place du dispositif 1 de l'invention est obtenue en introduisant d'abord le fil de guidage 12 à l'intérieur du vaisseau 14 au-delà de l'emplacement où se situent les dépôts 15 adhérant à la paroi interne 16 du vaisseau 14. Puis on introduit dans le vaisseau le cathéter 13 dans lequel est placée la canalisation 2, ladite canalisation 2 étant elle-même emmanchée sur le fil de guidage 12. Une fois que le bord avant 17 du cathéter 13 est arrêté par les dépôts 15, on effectue le retrait dudit cathéter 13 tout en bloquant en position la canalisation 2 de manière à dégager la partie avant 3 de la canalisation 2. Enfin on met en rotation la canalisation 2 de manière à effectuer le développement transversal des bandes 5 - 8 comme cela a été précédemment décrit. On déplace ensuite vers l'avant la canalisation 2 jusqu'à ce que les bandes 5 - 8 atteignent les dépôts 15 et les abrasent progressivement. Pendant cette opération l'opérateur peut déplacer vers l'avant puis d'avant en arrière, la canalisation 2 de telle sorte que les bandes 5 - 8 éliminent les dépôts 15. Dans le même temps, l'opérateur peut également agir sur la vitesse de rotation de la turbine d'entraînement pour parfaire l'efficacité de pénétration de l'outil rotatif de l'invention.

Sur la figure 2 on a représenté en fonctionnement un dispositif d'athérectomie selon l'invention qui comporte deux jeux successifs 18 et 19 d'éléments longiformes souples à développement transversal centrifuge.

S'agissant d'une canalisation du type de celle qui a été décrite dans l'exemple précédent, illustré par la figure 1, les deux jeux 18 et 19 sont obtenus en pratiquant dans la canalisation des entailles selon ce qui a été dit précédemment dans deux zones distinctes séparées par une partie intermédiaire 20 de canalisation. Cette partie intermédiaire 20 permet de garder solidaires les bandes du premier jeu 18 en complément de l'extrémité distale 9, ainsi que de garder solidaires les bandes du second jeu 19 en complément de l'extrémité proximale 10.

Lors de la mise en rotation de la turbine ou des moyens d'entraînement de la canalisation 2, il se produit progressivement le développement transversal du premier et du second jeux de bandes. Le premier jeu 18, le plus proche de l'extrémité distale 9 de la canalisation 2 est le premier en contact avec les dépôts 15. Une fois que le premier jeu 18 a érodé suffisamment les dépôts 15, il est possible en faisant avancer la canalisation 2 de faire entrer en action le second jeu 19 de manière à éliminer les dépôts restants, adhérant sur la paroi interne 16 du vaisseau 14.

Comme on peut le voir sur la figure 2, la tige de guidage 12 prend appui, dans sa partie antérieure 12a sur la paroi interne 16 du vaisseau 14 au niveau d'un coude 21. Ainsi la tige de guidage 12 est bloquée en position et il ne produit pas de battements lors de la rotation de la canalisation 2, battements qui seraient préjudiciables puisqu'ils pourraient d'une part entraîner une dégradation de la paroi interne 16 au niveau de l'extrémité avant 21 au niveau de la tige 12 et d'autre part perturber l'action du ou des outils rotatifs.

En ce qui concerne le dispositif d'athérectomie à deux jeux de bandes développables transversalement, il serait possible de ne faire agir dans un premier temps que le premier jeu 18, en laissant le second jeu 19 à l'intérieur du cathéter 13 puis dans un second temps de faire agir ce second jeu 19 après avoir déplacé la canalisation 2 dans le cathéter 13 de manière à ce que ce second jeu sorte dudit cathéter 13.

Dans le cas où il s'agit de fragmenter des thrombus, ceci peut être obtenu à grande vitesse par l'un des dispositifs qui vient d'être décrit. Par contre lorsqu'il s'agit d'abraser des dépôts durs dans des artères, il est souhaitable que la surface des éléments longiformes flexibles, à développement transversal centrifuge, soit abrasive. Ainsi il est possible d'obtenir une érosion progressive de ces dépôts durs.

Dans le cas d'une canalisation réalisée dans une matière plastique, par exemple en polytétrafluoréthylène, la surface abrasive peut être obtenue soit en collant de la poudre abrasive par exemple de la poudre de diamant, en surface de la partie avant 3 de la canalisation, soit en incluant lors du moulage de la canalisation 2, au niveau de la partie avant 3, de la poudre arasive dans la matière plastique.

Il est également préférable que le bord avant 11 de la canalisation 2 ait lui-même une surface abrasive, de manière à ce que ce bord avant 11 puisse avoir une action préliminaire vis-à-vis des dépôts 15, notamment dans le cas d'une obstruction importante du vaisseau.

L'invention n'est pas limitée aux modes de réalisation qui ont été décrits à titre d'exemples non limitatifs. En particulier le nombre d'éléments longiformes flexibles, constituant l'outil rotatif, peut être inférieur ou supérieur à quatre, étant généralement au moins de trois et au plus de huit.

Dans les exemples précités, le diamètre de l'outil rotatif lorsque les éléments longiformes sont en position rectiligne, étaient de 1,7 mm. Ce diamètre peut être encore plus faible puisque la seule contrainte est de pouvoir introduire dans la canalisation correspondante le fil de guidage, qui lui peut être très fin. Ainsi grâce au dispositif de l'invention il est possible d'intervenir dans des vaisseaux ayant un diamètre très restreint, par exemple de deux millimètres.

## Revendications

1. Dispositif d'athérectomie ou de thrombectomie comprenant une canalisation (2) de matériau plastique, portant à une extrémité distale (3) un organe rotatif composé d'éléments (5-8) longiformes flexibles ayant des extrémités proximales et distales, les extrémités distales étant unies l'une l'autre (9) et les extrémités proximales étant unies l'une l'autre (10), les éléments (5-8) longiformes flexibles étant développables transversalement pour atteindre un diamètre d'attaque par rapprochement axial des extrémités proximales (10) et distales (9), et comprenant des moyens d'entraînement en rotation pour actionner l'organe rotatif et de faire roter la canalisation (2) avec une vitesse de rotation supérieure à une certaine vitesse de rotation, **caracterisé** en ce que l'organe rotatif est constitue par la canalisation (2) présentant des entailles (4) longitudinales réparties symétriquement sur sa péripherie et délimitant les éléments (5-8) longiformes flexibles du matériau plastique de la canalisation (2), lesdites extrémités proximales (10) et distales (9) unies étant librement mobile axialement les une relativement aux autres et en ce que la flexibilité des éléments (5-8) longiformes est telle que avec la certaine vitesse de rotation les éléments (5-8) se dévéloppent transversalement au diamètre à attaque sous l'effet de la seule force centrifuge.

2. Dispositif d'athérectomie ou de thrombectomie selon la revendication 1, caractérisé en ce que la certaine vitesse de rotation est 30 000 tours/mn.

3. Dispositif d'athérectomie ou de thrombectomie selon la revendication 1 comprenant en plus un cathéter (13) pour recevoir la canalisation (2).

4. Dispositif d'athérectomie ou de thrombectomie selon la revendication 1 caractérisé en ce que les moyens d'entraînement consistent en une turbine, entraînée en rotation par un fluide comprimé et ayant une tubulure axiale dans laquelle est montée l'extrémité proximale de la canalisation (2.)

5. Dispositif d'athérectomie ou de thrombectomie selon la revendication 1 caractérisé en ce qu'il comporte un fil de guidage (12) rigide, pour être enfilé dans la canalisation (2).

6. Dispositif d'athérectomie ou de thrombectomie selon la revendication 1 caractérisé en ce que la canalisation (2) est réalisée en polytétrafluoréthylène.

7. Dispositif d'athérectomie ou de thrombectomie selon la revendication 1 caractérisé en ce que les éléments longiformes flexibles (5-8) de l'organe rotatif ont une surface abrasive.

8. Dispositif d'athérectomie ou de thrombectomie selon les revendications 1 et 7 caractérisé en ce que la surface abrasive est obtenue par inclusion d'une poudre abrasive dans le matériau plastique constitutif de la canalisation (2), au moins dans sa partie avant correspondant à l'outil rotatif.

9. Dispositif d'athérectomie ou de thrombectomie selon la revendication 1 caractérisé en ce que l'organe rotatif est composé de deux jeux successifs (18, 19) d'éléments longiformes flexibles.

10. Dispositif d'athérectomie ou de thrombectomie selon la revendication 9 caractérisé en ce que la longueur des éléments longiformes du jeu (18) le plus distal est inférieure à la longueur des éléments longiformes du jeu (19) le plus proximal.

## Claims

1. Atherectomy or thrombectomy device comprising a conduit (2) made of plastics material, bearing at a distal end (3) a rotary organ composed of flexible elongate elements (5 - 8) having proximal and distal ends, the distal ends being joined to each other (9) and the proximal ends being joined to each other (10), the flexible elongate elements (5 - 8) being transversally expandable to attain an attack diameter by axially moving the proximal (10) and distal (9) ends towards each other, and comprising rotational drive means to drive the rotary organ and to cause the conduit (2) to rotate with a rotational velocity greater than a certain rotational velocity, characterised by the fact that the rotary organ consists of the conduit (2) having longitudinal slits (4) symmetrically distributed about its periphery and defining the flexible elongate elements (5 - 8) of the plastics material of the conduit (2), the said joined proximal (10) and distal (9) ends being freely axially mobile relative to each other and by the fact that the flexibility of the elongate elements (5 - 8) is such that with the certain rotational velocity the elements (5 - 8) expand transversally to the attack diameter under the influence of centrifugal force alone.

2. Atherectomy or thrombectomy device as described in claim 1, characterised by the fact that the certain rotational velocity is 30 000 rpm.

3. Atherectomy or thrombectomy device as described in claim 1 also comprising a catheter (13) to receive the conduit (2).

4. Atherectomy or thrombectomy device as described in claim 1, characterised by the fact that the drive means consist of a turbine, rotated by a compressed fluid and having an axial tubulure in which the proximal end of the conduit (2) is mounted.

5. Atherectomy or thrombectomy device as described in claim 1, characterised by the fact that it includes a rigid guide wire (12) to be threaded into the conduit (2).

6. Atherectomy or thrombectomy device as described in claim 1, characterised by the fact that the conduit (2) is made of polytetrafluoroethylene.

7. Atherectomy or thrombectomy device as described in claim 1, characterised by the fact that the flexible elongate elements (5 - 8) of the rotary organ have an abrasive surface.

8. Atherectomy or thrombectomy device as described in claims 1 and 7, characterised by the fact that the abrasive surface is obtained by inclusion of an abrasive powder in the plastics material forming the conduit (2), at least in its forward part corresponding to the rotary tool.

9. Atherectomy or thrombectomy device as described in claim 1, characterised by the fact that the rotary organ consists of two successive sets (18, 19) of flexible elongate elements.

10. Atherectomy or thrombectomy device as described in claim 9, characterised by the fact that the length of the elongate elements of the most distal set (18) is less than the length of the elongate elements of the most proximal set (19).

## Patentansprüche

1. Vorrichtung zur Atherektomie oder Thrombektomie mit einem Rohr (2) aus Kunststoff, das an einem distalen Ende (3) ein drehbares Organ trägt, das zusammengesetzt ist aus langgestreckten flexiblen Elementen (5-9) mit proximalen und distalen Enden, wobei die distalen Enden miteinander verbunden (9) und die proximalen Enden miteinander verbunden (10) sind und die langgestreckten flexiblen Elemente (5-8) durch axiales Annähern ihrer proximalen (10) und distalen (9) Enden in Querrichtung ausbauchbar sind, um einen Arbeitsdurchmesser anzunehmen, und mit Drehantriebsmitteln zum Betätigen des drehbaren Organs und zum Drehen des Rohres (2) mit einer Drehgeschwindigkeit, die größer ist als eine bestimmte Drehgeschwindigkeit,
dadurch **gekennzeichnet,** daß das drehbare Organ dadurch gebildet ist, daß das Rohr (2) Längsschlitze (4) aufweist, die symmetrisch an seinem Umfang verteilt sind und die langgestreckten flexiblen Elemente (5-8) des Kunststoffmaterials des Rohres (2) begrenzen, daß die verbundenen proximalen (10) und distalen (9) Enden relativ zueinander frei axial beweglich sind und daß die Flexibilität der langgestreckten Elemente (5-8) derart ist, daß bei einer bestimmten Rotationsgeschwindigkeit die Elemente (5-8) sich allein durch Wirkung der Zentrifugalkraft in Querrichtung auf den Arbeitssdurchmesser ausbauchen.

2. Vorrichtung zur Atherektomie oder Thrombektomie nach Anspruch 1,
dadurch **gekennzeichnet,** daß die bestimmte Rotationsgeschwindigkeit 30000 Umdrehungen pro Minute trägt.

3. Vorrichtung zur Atherektomie und Thrombektomie nach Anspruch 1,
die ferner einen Katheter (13) zur Aufnahme des Rohres (2) aufweist.

4. Vorrichtung zur Atherektomie oder Thrombektomie gemäß Anspruch 1,
dadurch **gekennzeichnet,** daß die Antriebsmittel aus einer Turbine bestehen, die durch ein komprimiertes Fluid in Rotation versetzt wird und einen Rohrstutzen aufweist, in dem das proximale Ende des Rohres (2) gelagert ist.

5. Vorrichtung zur Atherektomie oder Thrombektomie nach Anspruch 1,
dadurch **gekennzeichnet,** daß sie einen steifen Führungsdraht (12) aufweist, der in das Rohr (2) einfädelbar ist.

6. Vorrichtung zur Atherektomie oder Thrombektomie nach Anspruch 1,
dadurch **gekennzeichnet,** daß das Rohr (2) aus Polytetrafluorethylen besteht.

7. Vorrichtung zur Atherektomie oder Thrombektomie nach Anspruch 1,
dadurch **gekennzeichnet,** daß die langgestreckten flexiblen Elemente (5-8) des drehbaren Organs eine abrasive Oberfläche haben.

8. Vorrichtung zur Atherektomie oder Thrombektomie gemäß den Ansprüchen 1 und 7,
dadurch **gekennzeichnet,** daß die abrasive Oberfläche erhalten ist durch Einschluß eines abrasiven Pulvers in das Kunststoffmaterial, aus dem das Rohr (2) besteht, mindestens in dessen vorderen, dem Drehwerkzeug entsprechenden Teil.

9. Vorrichtung zur Atherektomie oder Thrombektomie gemäß Anspruch 1,
dadurch **gekennzeichnet,** daß das drehbare Organ zusammengesetzt ist aus zwei hintereinander angeordneten Sätzen (18, 19) von langgestreckten flexiblen Elementen.

10. Vorrichtung zur Atherektomie oder Thrombektomie gemäß Anspruch 9,
dadurch **gekennzeichnet,** daß die Länge der langgestreckten Elemente des am weitesten distalen Satzes (18) kleiner ist als die Länge der langgestreckten Elemente des am weitesten proximalen Satzes (19).
